# EUROPÄISCHE PATENTSCHRIFT

(11) **EP 1 471 886 B1**
(45) Veröffentlichungstag und Bekanntmachung des Hinweises auf die Patenterteilung: **15.08.2007**
(21) Anmeldenummer: 03704402.1
(22) Anmeldetag: 15.01.2003
(51) Int. Cl.: A61K 9/127, A61P 25/24, A61P 9/00, A61P 19/02

(54) **VERFAHREN ZUR ERHÖHUNG DER WASSERLÖSLICHKEIT LIPOPHILER WIRKSTOFFE; HERSTELLUNG VON HOCHKONZENTRIERTEN WÄSSRIGEN ZUSAMMENSETZUNGEN DIESER WIRKSTOFFE; DERARTIGE PRODUKTE UND IHRE VERWENDUNG**
METHOD FOR INCREASING THE WATER-SOLUBILITY OF LIPOPHILIC, ACTIVE SUBSTANCES, PRODUCTION OF HIGHLY CONCENTRATED AQUEOUS COMPOSITIONS OF SAID ACTIVE SUBSTANCES, CORRESPONDING PRODUCTS AND THEIR USE
PROCEDE PERMETTANT D'ACCROITRE LA SOLUBILITE DANS L'EAU DE SUSBTANCES ACTIVES LIPOPHILES, PRODUCTION DE COMPOSITIONS AQUEUSES HAUTEMENT CONCENTREES DESDITES SUBSTANCES ACTIVES, PRODUITS CORRESPONDANTS ET LEUR UTILISATION

(30) Priorität: 31.01.2002 DE 10203923
(43) Veröffentlichungstag der Anmeldung: 03.11.2004
(73) Patentinhaber: KliniPharm GmbH, 60318 Frankfurt / Main (DE)
(72) Erfinder: BOGDANOVIC, Eva, 67251 Freinsheim (DE); GRZIMEK, Katja, 60385 Frankfurt/Main (DE); SCHATTON, Wolfgang, 65760 Eschborn (DE)
(74) Vertreter: Müller, Claudia
(86) Internationale Anmeldenummer: PCT/EP2003/000333
(87) Internationale Veröffentlichungsnummer: WO 2003/063830

(56) Entgegenhaltungen:
- WO-A-91/11993
- US-A- 4 877 561
- US-A- 5 716 638
- TOUITOU E ET AL: "Ethosomes - novel vesicular carriers for enhanced delivery: characterization and skin penetration properties" JOURNAL OF CONTROLLED RELEASE, ELSEVIER SCIENCE PUBLISHERS B.V., AMSTERDAM, NL, Bd. 65, Nr. 3, April 2000 (2000-04), Seiten 403-418, XP004190338 ISSN: 0168-3659

## Beschreibung

Die vorliegende Erfindung betrifft ein Verfahren zur Erhöhung der Löslichkeit lipophiler Wirkstoffe in wässrigen Systemen sowie hierauf basierend die Herstellung von hochkonzentrierten wässrigen Zusammensetzungen von lipophilen Wirkstoffen, ausgewählt aus Hormonen, Hormonanaloga, Terpenen, Imidazolen sowie Derivaten hiervon. Hierbei wird eine alkoholische Lösung des oder der Wirkstoffe mit vesikelbildenden Lipiden versetzt. Anschließend kann die so erhaltene Zusammensetzung mit einer Gel-haltigen Mischung umgesetzt werden. Dabei werden hochkonzentrierte Zubereitungen erhalten, welche als solche direkt als Gele topisch applizierbar sind oder gefriergetrocknet werden können. Im letzteren Fall werden Pulver erhalten, die je nach gewünschtem Anwendungsziel, weiter verarbeitet werden können. So können hieraus für die topische Applikation z.B. Cremes, Gele, Salben, Pflaster, für die orale Anwendung z.B. Lösungen, Kapseln, Tabletten, Granulat, besonders auch für die retardierte Freisetzung, sowie für die rektale und parenterale Applikation entsprechende Produkte sowie Implantate hergestellt werden.

Mit dem erfindungsgemäßen Verfahren ist es somit möglich, solche lipophilen Wirkstoffe in kosmetische oder pharmazeutische Zubereitungen in hoher Menge zu inkorporieren und darüber hinaus durch Gefriertrocknung in eine lagerstabile und besonders günstig transportable Form zu überführen, was mit bisherigen Zusammensetzungen dieser Art nicht möglich war.

Lipophile Wirkstoffe wie Terpene, Hormone, insbesondere Steroidhormone, oder Imidazole sind allgemein bekannt als schlecht löslich in wässrigen Systemen. Ferner sind solche Stoffe, insbesondere Hormone, bereits in kleinsten Mengen wirksam und werden oft nach kurzer Zeit metabolisiert und über Galle, Urin oder Faeces eliminiert. Sie müssen ständig nachgebildet werden, da eine Speicherung im Organismus nicht möglich ist. Der Hormonspiegel muß daher wegen der kurzen Wirkungszeit ständig dem jeweiligen Bedarf angepasst werden.

Steroidhormone sind körpereigene Wirkstoffe von besonderer Aktivität, die in Drüsen oder Gewebeteilen produziert werden und direkt in die Blut- oder Lymphbahn abgegeben werden. Ihre Aufgabe ist es, Stoffwechselvorgänge und die Tätigkeit einzelner Organe zu regulieren. Ihr Einfluß auf die Psyche ist in diesem Zusammenhang ebenfalls von großer Bedeutung.
Alle Steroidhormone werden im Organismus aus Cholesterin gebildet. Ihr Grundgerüst ist das tetracyclische Cyclopentano-perhydrophenanthren, auch Steran, genannt. Für das Verständnis ihrer physikalisch chemischen Eigenschaften ist es wichtig, daß sich alle Steroidhormone auf drei vom Steran abgeleitete Grundkörper zurückführen lassen, Oestran, Androstan und Pregnan mit 18, 19 oder 21, mit Wasserstoff, Alkyl-oder Hydroxy-substituierten C- Atomen. Folglich sind alle diese Verbindungen von Natur aus extrem lipophil. Sie sind im allgemeinen unlöslich in Wasser. Gute Lösungsmittel sind jedoch organische Solventien, wie z.B. Chloroform, Petrolether, Hexan, Ether, Dioxan, Aceton, Methylbutylketon, Alkohole, pflanzliche und mineralische Öle [vergl. z.B.: The Merck Index, An Encyclopedia of Chemicals, Drugs and Biologicals, 11 th Edition, 1989].
Hormonanaloga sind Substanzen mit hormonähnlicher Wirkung. Hierzu gehören insbesondere auch im Merck Index unter der therapeutischen Kategorie der Östrogene und Antiöstrogene, Androgene, Progestogene oder Anabolika geführten Wirkstoffe.
Terpene sind Wirkstoffe mit Isopren als chemischen Gründgerüst und umfassen acyclische und Mono-, Di-Tri-oder Tetracyclische Verbindungen, welche wie die beschriebenen Hormone meist schwerlöslich in Wasser sind und daher bezüglich ihres Löslichkeitsverhaltens analoge Eigenschaften aufweisen.
Gleiches gilt auch für Imidazole. Diese stellen 1,-3-Di-Stickstoff-substituierte ungesättigte Cyclopentane dar, welche insbesondere in der 2, 4 und 5-Position des Ringes oder am N1-Stickstoff substituiert sein können.

Bisher werden solche Wirkstoffe meist in Tablettenform verabreicht, welche langsam im Intestinaltrakt oder aus dem Gewebeimplantat freigesetzt werden. Dabei sind die Tabletten mit Hilfsmitteln und Zusatzstoffen versetzt, welche einerseits die Freisetzung ermöglichen und andererseits zur Stabilisierung erforderlich sind. Insbesondere bei Hormonen besteht die Schwierigkeit, eine ausreichende Menge an Wirkstoff zu inkorporieren.
So weisen pharmazeutische Zubereitungen von Steroidhormonen für die topische Applikation, welche gegenwärtig als übliche Emulsionen bzw. Wirkstoffsuspensionen vom Typ Wasser in Öl oder umgekehrt darstellen, in denen die Wirkstoffe meist in fein kristalliner Verreibung eingearbeitet sind, eine Wirkstoffkonzentrationen von1%, meist aber deutlich weniger, auf.
Injizierbare Präparate, wie Testoviron Depot® oder Gynodian Depot® enthalten die Hormone in öliger Suspension in einer Menge bis zu 10%. Nachteilig ist hier, dass solche Produkte nur intramuskulär appliziert werden können, eine Ausbildung von Microkristallen nicht unerwünscht ist sondern zur Depotwirkung beiträgt und klare Lösungen nicht lange haltbar sind.

Das Präparat AndroGel® der Firma Unimel Pharmaceuticals [vergl. ANDROGEL.COM] wird als unsichtbares Gel mit einem Gehalt von 1% Testosteron beschrieben. Demnach ist eine höhere Menge an Hormonwirkstoff in einem solchen Fall nicht möglich.

Im NRF, Neues Rezeptur-Formularium, 17. Ergänzung 2000, GOVI-Verlag, Eschborn, findet sich eine herkömmlich hergestellte 2%ige hydrophile Testosteronpropionat-Creme (NRF 25.4), bei der allerdings auf die Gefahr eines möglichen Kristallwachstums hingewiesen wird.

Es besteht daher Bedarf an einem einfachen und wirtschaftlichen Verfahren zur Verbesserung der Löslichkeit, insbesondere in Wasser, solcher lipophiler hochwirksamer Stoffe, um Zusammensetzungen bereitzustellen, welche den oder die lipophilen Wirkstoffe in hoher Menge enthalten, stabil sind, im wesentlichen frei von Zusatzstoffen sind und dennoch in gewünschter Weise freisetzen können.

Aufgabe der vorliegenden Anmeldung ist es daher, eine produktorientierte Methode für Zubereitungen von lipophilen Wirkstoffen zu entwickeln, welche einfach und effektiv in guten Ausbeuten zu einem Produkt mit hohem gelösten Wirkstoffanteilen führt, wobei das Auskristallisieren der Wirkstoffe auch unter extremen Lagerbedingungen, nämlich in einem Temperaturbereich von -30° Celsius bis 50° Celsius, verhindert werden soll.
Gleichzeitig sollen die Produkte kostengünstig, praxisnah und hautverträglich so konserviert sein, so dass die Zubereitungen auch von Patienten, die zu Allergien neigen, gut vertragen werden.
Ferner sollen Zubereitungen hergestellt werden, die den Wirkstoffen eine optimale Bioverfügbarkeit verleihen.

Diese Aufgabe wird erfindungsgemäss gelöst, indem man den oder die lipophilen Wirkstoffe in gesättigter bis übersättigter alkoholischer Lösung mit Vesikelbildnern (Liposomen), versetzt. Diese übersättigten Wirkstofflösungen können anschließend in wässrige Lösungen, enthaltend Gelbildner, eingearbeitet werden. Dabei erhält man überraschenderweise Zubereitungen mit Konzentrationen von weit mehr als 1 % an gelösten Wirkstoffen, aus denen diese auch unter extremen Bedingungen nicht auskristalliseren.
Überraschenderweise werden bei dieser Vorgehensweise Gele in der Art "nicht Newton'scher Flüssigkeiten" erhalten. Es zeigte sich nämlich, dass die Viskosität der erfindungsgemäß hergestellten Gele bei Werten um 0° Celsius paradoxervveise so stark erniedrigt ist, dass wasserklare dünnflüssige Systeme erhalten werden, die beim Raumtemperatur ihre typische hohe Viskosität wieder annehmen. Möglicherweise liegen aufgrund der erfindungsgemäßen Herstellungsweise Wirkstoffkomplexe vor, welche ein derartiges physikalisches Verhalten bedingen, das überraschenderweise auf einfache und wirtschaftliche Weise einerseits zu der erhöhten Löslichkeit der Substanzen und andererseits zu einer wesentlich verbesserten Stabilität führt.

Falls gewünscht, können die erhaltenen Gele auch gefriergetrocknet werden. Nach der Gefriertrocknung dieser Lösungen erhält man pulvrige Wirkstoffkomplexe. Diese lassen sich zu üblichen festen oder halbfesten oder flüssigen pharmazeutischen Zubereitungen weiterverarbeiten. In Wasser oder Alkohol-Wassergemischen quellen diese Wirkstoffkomplexe wiederum zu stabilen Gelen, auf. In ihnen liegen die anfänglich eingearbeiteten Wirkstoffe nicht kristallin, sondern überraschend wiederum in gelöster Form in der jeweils gewünschten Konzentration vor. Die erfindungsgemäß hergestellten WirkstoffKomplexe können, sofern sie nicht direkt als Gel eingesetzt, sondern gefriergetrocknet werden, auch in andere gewünschte Formulierungen, z.B. Creme, Salbe, Injektion mit den dafür üblichen Komponenten in hoher Konzentration eingearbeitet werden. Sie liegen dann nach dem Umsetzen mit der Flüssigkeit wieder in gelöster Form vor, und nicht suspendiert. Damit werden stabile, insbesondere lagerstabile Produkte mit hoher Wirkstoffkonzentration erhalten.

Die vorliegende Erfindung betrifft somit ein Verfahren zur Verbesserung der Wasserlöslichkeit von lipophilen, insbesondere pharmazeutisch wirksamen Substanzen, ausgewählt aus Hormonen, Hormonanaloga, Terpenen, Imidazolen, Harzen und Derivaten hiervon, das dadurch gekennzeichnet ist, dass man die lipophile(n) Substanz(en) in eine mindenstens äquivalente Menge eines Lösungsmittels, bezogen auf das Gewicht der Wirksubstanz, ausgewählt aus einund mehrwertigen Alkoholen und Mischungen hiervon bei 20° bis 70°C unter Hinzufügen von 0,1 -20 Gew.%, bezogen auf die Wirksubstanz, eines oder mehrerer vesikelbildender Lipide einarbeitet.
Vorzugsweise wird bei erhöhter Temperatur gearbeitet. Vor dem Abkühlen kann dann die wässrige Lösung eines Gelbildners, wie Polyacrylat oder Hydroxypropylmethyl- -propyl- oder -ethylcellulose oder Agarose, Kollagen oder andere polymere Kohlehydrate oder Proteine, z.B. als 0,5 bis 10%ige Lösung, zugesetzt werden. Beim Abkühlen liegen dann Gele vor mit hohem Gehalt an lipophilen Wirkstoffen.
Insbesondere sind die wirksame(n) Substanz(en) ausgewählt aus Hormonen, vor allem Steroidhormonen, Terpenen, Imidazolen und Derivaten hiervon. Ganz besonders bevorzugte Wirkstoffe sind Hormone, vor allem Steroidhormone, Terpene und Derivate hiervon. Am meisten bevorzugt sind Terpene und Hormone und hierunter insbesondere Hormone, speziell Steroidhormone.

Zu den Derivaten der Hormone gehören vorzugsweise die Ester wie Acetate, Propionate, oder auch Butyrate, Lactone oder andere chemische Derivate

Als Terpene bzw. Derivate hiervon eignen sich vor allem solche als Wirkstoffe bereits bekannte Verbindungen, wie z.B. Vitamin A, Carotinoide wie β-Carotin oder Retinoide, Ginkgo-Extrakt, Avarol, Avaron, Campher, Terpenketone und Terpenalkohole wie Menthol, Terpineol, Borneol, Farnesol, Menthon oder Squalenen. Es handelt sich dabei allgemein um Riechstoffe (ätherische Öle), Farbstoffe und Pflanzenextrakte mit bestimmten, insbesondere durchblutungsfördemden, entzündungshemmenden, antimikrobiell, zytoststisch wirksamen und allgemein das psychsiche Wohlbefinden beeinflussende Eigenschaften. Besonders bevorzugt sind Carotinoide, Ginkgo-Extrakt, Avarol und Avaron, Terpenalkohole.. Ganz besonders bevorzugt sind Avaron, Avarol, Carotinoide und Derivate hiervon.

Die Imidazole gemäß vorliegender Erfindung sind allgemein 1,-3-Di-Stickstoff-substituierte ungesättigte Cyclopentane, welche insbesondere in der 2, 4 und 5-Position des Ringes oder am N1-Stickstoff substituiert sein können mit C₁₋₁₀-Alkyl, C₁-10-Alkoxy, C₃₋₈-Cycloalkyl, Halo, Nitro, Cyano, Hydroxy oder Aryl, insbesondere Phenyl, substituiert mit den vorstehend genannten Gruppen, wie z.B. im Falle des Metronidazols, einem 1-(2-hydroxyethyl)-2-methyl-5-nitro-imidazol, welches besonders bevorzugt ist.

Insbesondere die' N1- Sulfonamid- oder N1-Phenyl-Sulfonamid-substituierten Verbindungen mit einem Phenylrest in 3- oder 5 Stellung sind als entzündungshemmende, antibakteriell wirksame Stoffe bekannt, vgl. Econazol, Ketoconazol oder andere bekannte Imidazol-Fungistatika. Diese Verbindungen sind ebenfalls bevorzugt.
Bekannte Abkömmlinge von Imidazolen sind beispielsweise Aminosäuren ( Histidin, Histamin), antibakterielle oder fungistatische Mittel (Metronidazol, Clotrimazollsoconazol, Bifonazol, Tioconazol, u.a. vergl. Merck Index). Die antibakteriellen und fungistischen Imidazole sind bevorzugt.

Bei Terpenen und Imidazolen liegen je nach Substituentenmuster die Verbindungen als Ester, Alkali- oder Erdalkali- oder Ammoniumsalze bzw. Amide vor,
wie Natrium-/KaliumlAmmonium/Magnesium/Calcium etc-Salze, Ester und Aminocarbonylverbindungen (Amide), je nach Substitutionsmuster.

Zu der Gruppe der Hormone gehören die Peptidhormone, z.B. Schilddrüsen-, Nebennierenmark- (NNM), Hypothalamus- und Hypophysenhormone wie sie aus üblichen Lehrbüchern der Pharmakologie, z.B. E. Mutschler et. al., Arzneimittelwirkungen, DAV Verlag 2001 bekannt sind, Adenosinphosphate (Adenosinphosphorsäuren, Adenosinphosphorsäureester), Phosphorsäureester des Adenosins.

Insbesondere gehören hierzu auch die besonders bevorzugten Steroidhormone.

Dabei handelt es sich um Hormone aus der Nebennierenrinde (NNR) und aus den männlichen und weiblichen Sexualorganen. Diese sind klein, fettlöslich und können die Membran durchdringen und wirken somit aus dem Zellinneren heraus.

Besonders bevorzugt sind Estrogene wie 17β-Estradiol, Estradiol , Estradiolester, Estriol , Estriol-konjugiertes Estrogen, Ethinylestradiol, Dienestrol sowie Methestrol sowie weitere bekannte Östrogene und Antiöstrogene.

Ebenso bevorzugt sind Progesterone und Abkömmlinge hiervon wie Progesteron, Progesteron-C-21 Steroide wie Cyproteronacetat , Dydrogesteron, Medrogeston, Medroxyprogesteronacetat, Megestrolacetat, Promegeston, Nortestosteronabgeleitete(C-1 9 Steroide) wie Levonorgestrel, Norethisteron, Norethisteronacetat, Norgestrel sowie andere therapeutisch bekannte Progestogene, vergl. Merck Index.

Ferner gehören hierzu Androgene wie Testosteron und Testosteronester wie z.B. die Acetate, Propionate oder Methyltestosteron, Dihydrepiandrosteron (DHEA) sowie andere therapeutisch bekannte Androgene, vergl. z.B. Merck Index.

Ganz besonders bevorzugt sind Testosteron, Estradiol, Estriol, Dihydroepiandrosteron (DHEA), Progesteron, sowie Mischungen hiervon und deren pharmazeutisch verträgliche Ester, insbesondere die Acetate und Propionate.

Zu der Gruppe der Hormonanaloga gehören therapeutisch bekannte chemische Derivate, vergl. Merck Index ..
Allgemein sind Derivate von Hormonen und Hormonanaloga die Ester, Lactone oder Redoxprodukte der entsprechenden Verbindungen.

Zu der Gruppe der Harze gehört insbesondere Propolis.
Das erfindungsgemäße Verfahren wird bevorzugt bei Temperaturen von 30°C bis 60°C, vor allem 30°C bis 50°C durchgeführt.
Je nach Wirkstoff kann eine pH-Wert- Anpassung auf bekannte Weise erfolgen, insbesondere nach Umsetzen mit Gelbildnern wie nachfolgend beschrieben.
Als Alkohol(e) sind bevorzugt einwertige C₁-C₁₂ -aliphatische oder C₁-C₁₂ aliphatisch-aromatische einwertige oder C₁-C₁₂ zweiwertige, insbesondere aliphatische, Alkohole oder Mischungen hiervon.
Insbesondere geeignet sind einwertige C₁-C₈-aliphatische Alkohole, vor allem aber C₁-C₄-aliphatische Alkohole, wie insbesondere Ethanol, 1-Propanol, 2-Propanol, 1-, 2-, oder 3-Butanol, 1-,2-oder 3-Pentanol, oder 1-,2-,3-,4- oder tert.-Butanol; ferner sind auch Hexanol, Oktanol geeignet sowie vor allem C₁-C₈, insbesondere C₁-C₄- zweiwertige Alkohole wie Propandiole, Butandiole oder Ethylenglykol, Propylenglykol oder Mischungen hiervon mit den genannten aliphatischen, insbesondere C₁₋₈- Alkoholen.
Geeignete aliphatisch-aromatische Alkohole sind z.B. Benzylalkohol, Thymol oder ihre Derivate.
Unter den 2-wertigen Alkoholen ist insbesondere Propylenglykol, aber auch Ethylenglykol bevorzugt.
Besonders bevorzugt sind einwertige Alkohole der oben beschriebenen Art, wie C₁₋₈- aliphatische, vor allem Ethanol sowie Mischungen hiervon mit Propylenglykol, insbesondere im Verhältnis 1:0,1 bis 5:1.

Die Menge an eingesetztem Alkohol oder Mischungen hiervon entspricht wenigstens der Gewichtsmenge an eingesetzter Wirksubstanz. Vorzugsweise entspricht sie der 1 bis 10-fachen, insbesondere 1,5 bis 6-fachen und ganz besonders der 2 bis 4-fachen Menge, bezogen auf das Gewicht der Wirksubstanz.

Das oder die vesikelbildenden Lipide sind bevorzugt ausgewählt aus Lecithin, Cholesterin, Phosphatidylcholin oder -serin, Squalenen, Sphingolipiden wie Sphingosin oder Sphingomyelin oder Mischungen hiervon, z.B. im Verhältnis von 1:1 bis 1:20 oder 1:1:1 bis 1:20:20 etc.. Eine besonders geeignete Mischung ist Lecithin mit Cholesterin, z.B. im Verhältnis von 1:1 bis 1:15, insbesondere 1:10.
Das oder die vesikelbildenden Lipide werden vorzugsweise in einer Menge von 0,5-15 Gewichtsprozent, bezogen auf die gewählte Wirkstoffmenge, insbesondere 1-10%, eingesetzt.
Erfindungsgemäß erfolgt eine hohe Löslichkeit der genannten Wirkstoffe erst durch Umsetzen mit dem oder den Alkoholen. Ohne diesen Zusatz, d.h. die alleinige Zugabe des Vesikelbildners in den angegebenen geringen Mengen bewirkt eine derartige Löslichkeitserhöhung nicht.

Somit können erfindungsgemäß wasserhaltige Gele mit einem Gehalt von 2 bis 90%, bevorzugt 2,5 bis 90%, an lipophilen Wirksubstanzen, hergestellt werden, indem man wie beschrieben, die Wirksubstanz(en) in eine mindenstens äquivalente Menge, bezogen auf das Gewicht, eines Lösungsmittels, ausgewählt aus ein- und mehrwertigen Alkoholen und Mischungen hiervon, bei 20° bis 70°C unter Hinzufügen von 0,1 -20Gew.%, bezogen auf das Gewicht der Wirksubstanz(en) eines oder mehrerer vesikelbildender Lipide einarbeitet, und sodann die 1 bis 10-fache Menge (Gewichtsprozent), bezogen auf das Gewicht der alkoholischen Lösung des lipophilen Wirkstoffs, einer wässrigen Lösung, enthaltend 0,1-5% eines Gelbildners, ausgewählt aus Polyacrylaten, Hydroxypropylmethyl- Hydroxyethyl- oder -propylcellulose, Agarose oder anderen polymeren Kohlehydraten hinzufügt und die Mischung anschließend, sofern erforderlich, abkühlt.
Gegebenenfalls können pH-Wert-Regulatoren bekannter Art wie nachstehend für weitere Formulierungsarten beschrieben, beigefügt werden.
Bevorzugt wird als Gelbildner ein Polyacrylat, wie z.B. Carbopol eingesetzt.
Die Hydrogele können hergestellt werden durch Einsatz von Gelbildner und Wasser, wobei erstere insbesondere ausgewählt werden aus natürlichen Produkten wie Cellulose-Derivaten, wie Zellulose-Ester und -Ether, z.B. Hydroxyethyl- Hydroxypropyl-Derivate, z.B. Tylose, oder auch aus sythetischen Produkten wie Polyacrylsäure-Derivate, wie Carbopol oder Carbomer, wie z.B. P934, P940, P941.
Sie können nach bekannten Vorschriften, die in den gängigen Arzneibüchern beschrieben sind wie z.B. DAB10 oder Europäisches Arzneibuch, aktuelle Ausgabe, z.B. 2000, aus alkoholischen Suspensionen durch Basenzusatz für die Gelbildung hergestellt bzw.polymerisiert werden.

Insbesondere beträgt die Menge an eingesetzter Wirksubstanz 2 -70%, vor allem 2,5 bis 70%, oder 5-60% und insbesondere 10-50%. Ganz besonders bevorzugt sind Produkte mit Anteilen von 11-90%, bevorzugt 11-70% und insbesondere 11-50% Wirksubstanz.

Auf diese Weise wird ein wasserhaltiges Produkt erhalten, welches man in an sich bekannter Weise gefriertrocknen kann bei Temperaturen unter -10°C und sodann ein lagerbeständiges Wirkstoff-haltiges Pulver erhält, welches 2 bis 90, oder bevorzugt 2,5 bis 90% Gewichtsprozent bzw. die anderen oben angegebenen Mengen an lipophilem(n) Wirkstoff(en)s, ausgewählt aus Hormonen, **Steroidhormonen,Terpenen, Imidazolen, Harzen und pharmazeutisch** verträglichen Derivaten hiervon aufweist.
Bevorzugt sind als Wirkstoff(e) Hormone, Terpene, Imidazole und Derivate hiervon enthalten. Die Mengen an Wirkstoff ist bevorzugt wie oben angegeben.

Die auf die erfindungsgemässe Weise erhaltenen Wirkstoffprodukte, die auch nach dem gefriertrocknen wieder in hoher Konzentration gelöst werden können aufgrund der nach dem erfindungsgemäßen Verfahren erzeugten Komplexstruktur, finden breite Anwendung in der Pharmazie, Medizin, Kosmetik und Lebensmittelchemie, wie z.B. in der topischen, cutanen, subcutanen, intramuskulären, enteralen oder parenteralen Therapie, insbesondere der Hormonsubstitution, als auch als Implantat.
Sie können daher verwendet werden zur Herstellung eines kosmetischen, medizinischen oder pharmazeutischen Mittels für die topische, parenterale intra- / sub-cutane, intravenöse, intramuskuläre, orale oder rektale Anwendung, bzw. als Implantat oder auch als Pflaster, der mit dem erfindungsgemäß hergestellten Produkt ausgestattet ist. Dazu kann entweder das Gel selbst eingesetzt werden oder das gefriegetrocknete Pulver gelöst werden im gewünschten Medium, wie in Wasser, Alkohol, insbesondere Ethanol, Propanol, Ethylenglykol, Propylenglykol oder Mischungen hiervon, physiologischer Kochsalzlösung oder anderen geeigneten pharmazeutisch zulässigen Trägern oder Mischungen hiervon, wobei die jeweils dann gewünschte Konzentration eingestellt werden kann. Mit flüssigen Trägern werden Gele, Injektionen und Lösungen erhalten. Bei Einsatz von geeigneten Fettphasen können auf übliche Weise Cremes oder Salben hergestellt werden. Als Fettphasen eignen sich z. B. natürliche Öle oder Wachse wie Mandelöl, Pfirsischkernöl, Weizenkeimöl, Sonnenblumen- Jojobaöl oder Olivenöl oder Kohlenwasserstoffe wie flüssige Paraffine, (Paraffin Perliquidum) isoparaffine, Dioctylcyclohexane, Isohexadecane, oder Squalen, Squalan, insbesondere auch Oleylalkohol, Octyldodecanol (Eutanol^{®} G);Fettsäureester, z.B. lsopropylfettsäureester (Palmitat, Myristat, Isostearat, Oleat),oder ähnliche; ferner Triglyceride wie Caprytic/Capric Triglyceride (Miglyol^{®} 810, 812) , Propylene Glycol Dicaprylatel Dicaprate (Miglyol^{®} 840); oder Silikonöle und - wachse.
Besonders bevorzugte Öl-Komponenten sind flüssige Paraffine und natürliche Öle.

Emulgatoren werden bevorzugt nicht eingesetzt. Falls sie jedoch erwünscht sein sollten, könnten hierfür bekannte und gängige Produkte eingesetzt werden, z.B. W/O- oder O/W- Emulgatoren. Zu den W/O-Emulgatoren gehören z.B. Cetylstearylalkohol, Glycerinmonostearat oder Span ® 85 (Sorbitantrioleat) oder andere pharmazeutisch übliche Sorbitanderivate, wie sie in den gängigen **Pharmakopöen zu finden sind, oder analoge Produkte wie Arlacel 85, Span 65** (Sorbitantristearat) Arlacel 65, Propylenglykolmonostearat, Sorbitanmonooleat, Span 40, Arlacel 40, Span 20 oder andere pharmazeutisch-technologisch übliche Analoga.
Zu geeigneten O/W-Emulgatoren gehören Polyethylenglycole mit geeignetem Ethoxylierungsgrad; wie z.B, wie z.B. Polyoxyethylenglykol-400-monostearat, oleylether, -monolaurat,-sorbitanmonolaurat, -sorbitantristearat).

Zur Herstellung von Salben verwendet man bevorzugt Kohlenwasserstoffe und Wollwachsalkohole.

Die Mengen an Fett, ggf. Emulgator sind allgemein bekannt und z.B. beschrieben in DAB10 oder dem aktuellen Europäischen Arzneibuch.

Auf die beschriebene Weise können Mittel bereitgestellt werden, welche den an sich zunächst schwer inkorporierbaren Wirkstoff in stabiler gelöster Form und überraschenderweise hoher Menge aufweisen.

Die Menge des verabreichten Mittels und der Dosierungsplan zur Behandlung eines krankhaften'oder vorzubeugenden Zustandes mit dem beschriebenen Mittel hängen von einer Vielzahl von Faktoren ab, einschließlich dem Alter, Gewicht, Geschlecht und medizinischem Zustand des Patienten, der Schwere der Erkrankung, der Verabreichungsroute und der Häufigkeit der Verabreichung und der jeweiligen verwendeten Verbindung, und kann deshalb weitestgehend schwanken. Bei der Herstellung der Formulierungen können ggf. ein oder mehrere Adjuvantien, die sich für die angegebene Verabreichungsroute eignen, hinzugefügt werden. Wenn per os verabreicht wird, kann das beschriebene Trockenprodukt auch vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor-und Schwefelsäuren, Gelatine, Akaziengummi, Natriumalginat, Glykole Polyvinylpyrrolidon und/oder Polyvinylalkohol und dann tablettiert oder verkapselt werden zur einfachen Verabreichung. Formulierungen für die parenterale Verabreichung können in Form von wässrigen oder nicht-wässrigen isotonischen sterilen Injektionslösungen oder Suspensionen vorliegen. Diese Lösungen und Suspensionen können hergestellt werden aus den erfindungsgmäßen Pulvern oder Granulaten mit einem oder mehreren der Adjuvantien, wie sie genannt wurden für die Verwendung in den Formulierungen für orale Verabreichung. Die Mittel können gelöst sein in Wasser, Polyethylenglykol, Propylenglykol, Ethanol, Maisöl, Baumwollsamenöl, Erdnußöl, Olivenöl, Sesamöl, Benzylalkohol, Natriumchlorid und/oder verschiedenen Puffern ( Stickstoff-/Phosphat-/Sulfat-/Carbonatpuffer ). Bei Wasser/Alkohol als Lösungsmittel entstehen wieder die bereits beschriebenen Gele. Es können auch pH-Regulatoren wie HCl, Phosphorsäurepuffer, NaOH, EDTH-Na, oder NH40H, K2C03 oder andere vorliegen. Ferner können Parfüm-, Farbstoffe, zu denen die genannten Terpene auch gehören oder UV-Filter wie Titandioxid, oder Konsistenzgeber in topisch applizierbaren Produkten enthalten sein.
Allerdings kann überraschenderweise aufgrund der besonderen Herstellungsweise der Wirkstoffkomplexe auf Stabilisatoren und Konservierungsmittel im wesentlichen verzichtet werden.

Die Menge an Zusatzstoffen beträgt vorzugsweise 0-10, insbesondere 0,1-5 Gewichtsprozent insgesamt.
Daneben kann das erfindungsgemäße Gel auch als solches auf geeignete Träger aufgebracht werden und als Implantat zur Wirkstofffreigabe verwendet werden. Träger und Vorgehensweise hierfür sind bekannt und beschrieben in Lehrbüchern der pharmazeutischen Technologie, z.B. Hagers Handbuch der Pharmazeutischen Praxis.
Es zeigte sich dabei, dass der auf welche Weise auch immer verabreichte Wirkstoff langsam, also protrahiert in die Blutbahn abgegeben wird und dort einen Ausgleich einer Wirkstoff-, insbesondere Hormonstörungslage, bedingt. So kann je nach eingesetztem Wirkstoff eine Hormonmangelsituation, insbesondere altersbedingt, wie Depression, Hautalterung, Muskel reduktion, etc. oder auch ein entzündlicher Zustand, Artheriosklerose, Störungen des zentralen Nervensystems, Kardiovaskuläre Erkrankungen, insbesondere auch als Vorbeugung vor diesen Dysfunktionen erfolgen.
Mittels der Terpen-Wirkstoffe oder der Imidazole können insbesondere entzündliche Erkrankungen wie Arthritis, bakterielle und mikrobielle Entzündungen, rheumatische Erkrankungen sowie degenerative Veränderungen des Bewegungsapparates behandelt oder vorgebeugt werden.

Ganz besonders bevorzugt sind topische Anwendungen in Form von Cremes, Salben Gelen, vor allem der Einsatz der Gele mit Hormonen, insbesondere Steroidhormonen oder Terpenen bzw. Derivaten oder Mischungen hiervon. Auch Produkte mit Imidazolen oder Mischungen hiervon mit Hormonen, Hormonanaloga, Terpenen in topischer Form sind bevorzugt.
Eine besonders bevorzugte Form sind die gefriergetrockneten Produkte, und unter diesen jene, enthaltend Hormone, oder Steroidhormone. Weiterhin bevorzugt sind auch Injektionen.

Mit dem erfindungsgemässen Verfahren ist es somit möglich, in hohen Ausbeuten Lösungen von lipophilen Wirkstoffen, insbesondere Steroidhormonen, in wässrigen Systemen oder in einer leicht wasserlöslichen Form ohne aufwendige Verfahrensschritte zu erhalten.
Das Verfahren eignet sich insbesondere für ein industrielles Scaling - up. Die Ausbeute an Wirkstoffprodukt ist quantitativ.

Dieses neue Verfahren zur Herstellung der hydrophilen Wirkstoffkomplexe ermöglicht somit die Produktion von hydrophilen Wirkstoff-, insbesondere Steroid-Rohstoffen, die im Unterschied zu bekannten Verfahren und analogen Handelspräparaten die Darstellung hochkonzentrierter stabiler Lösungen, insbesondere von Hormonen in wässrigen therapeutischen Systemen erst praktikabel erreichen lässt.

Die Erfindung wird anhand der nachfolgenden Beispiele näher erläutert.

### Beispiel 1

### Verfahren zur Darstellung von hochkonzentrierten wässrigen Steroidhormonlösungen

Zur Darstellung von 5kg einer 10%igen Lösung von kristallinem Testosteron oder Testosteronpropionat in einem wässrigen Polyacrylatgel werden 500g des Steroidhormons unter Rühren und kontrollierter leichter Erwärmung auf ca. 50°Celsius durch langsame Zugabe einer Lösung von 1 Gewichtsteil absoluten Ethanols und 1 Gewichtsteil Propylenglycol (1:1) vorsichtig gelöst. Hierzu werden im allgemeinen maximal 500g dieses Lösungsmittelgemisches eingesetzt. [Beim Abkühlen einer solchen Lösung kristallisiert das Hormon sehr rasch in den bekannten großen Kristallnadeln aus].
In der Wärme wird anschließend sofort wird ein Lipsomenkonzentrat mit Liposomen, z.B. aus 1g Cholesterin und 10g Lecithin, zugesetzt und der Ansatz mit wässrigem Carbopolgel, z.B aus Carbopol 940 oder 980, auf 5kg aufgefüllt.

Das Carbopolgel wird separat hergestellt aus 5kg gereinigtem Wasser unter Zusatz von 45 bis 75g Carbopol je nach Typ des verwendeten Carbopols und vorsichtig mit konzentrierter NaOH-Lösung neutralisiert.

Man erhält eine klare, in einem weiten Temperaturbereich äußerst stabile Lösung des Hormons in dem Polyacrylatgel mit einer relativ geringen Alkoholkonzentration, aus der die Testosteronsubstanz überraschenderweise nicht mehr auskristallisiert.

### Beispiel 2

### Verfahren zur Darstellung von Steroidhormonkomplexen durch Gefriertrocknung

Die nach Beispiel 1 hergestellten hochkonzentrierte wässrigen Steroidhormon-Gele werden in handelsüblichen Geräten dem Stand der Technik entsprechend gefriergetrocknet.
Die Ausbeute an Steroidhormonkomplexen ist quantitativ. Die so gewonnen kristallinen Komplexe lassen sich in Gegenwart z.B. physiologischer alkoholisch wässriger Lösungsmittelgemische zu hochkonzentrierten Gelen mit den Steroidhormonen in gelöster Form rekonstituieren.

### Beispiel 3

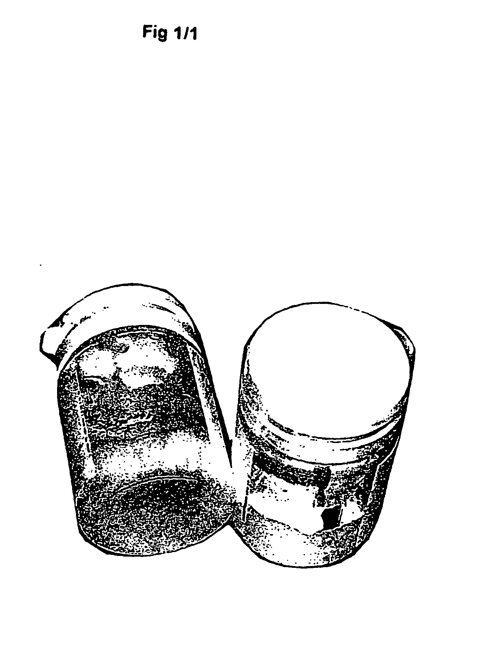

in Fig. 1 ist ein gemäß Beispiel 2 erhaltenes gefriergetrocknetes Produkt und das daraus nach Zugabe der wässrigen Mischung aller flüssigen Bestandteile wiederhergestellte Gel dargestellt.

### Beispiel 4

### Hormonsubstitution mit einem nach Beispiel 1 hergestellten hochkonzentrierten wässrigen Testosterongel

Erfahrungen mit einem nach Beispiel 1 hergestellten wässrig-alkoholischen Testosteron-Liposomengel wurden bisher über mehr als 1000 Patienten-Monate gesammelt. Hierzu wurde ein Gel, hergestellt wie in Beispiel 1 beschrieben, enthaltend 10g Testosteron in 100g Gelgrundlage, den Patienten täglich in einer Menge von 0,1 g auf die Unterarmhaut aufgetragen. Der Testesteronspiegel wurde im Blut auf bekannte Weise ermittelt. Dabei konnte bei ausgezeichneter Hautverträglichkeit des Gels der anfangs an der Grenze des unteren Normbereichs liegende Testosteronwert in den mittleren physiologischen Normbereich angehoben werden. Die Patienten berichteten übereinstimmend über Verbesserungen der Lebensqualität, im Bereich von Müdigkeit, Depression, abnehmender Muskelmasse und Kraft.

### Beispiel 5

### Hormonsubstitution mit Östrogenen und Gestagenen

Auf die gleiche, wie im Beispiel 4 beschriebenen Weise wurde auch die transdermale Hormonsubstitution mit Östrogenen oder Gestagenen untersucht. Zur Anwendung kamen wässrige Polyacrylatgele mit Progesteron [10%ig], Estradiol [10%ig], Estriol [5%ig], DHEA [20%ig].

Nach 4 wöchiger dermaler Applikation waren die bei allen Patienten zuvor im unteren Normbereich gelegenen Hormonspiegel in den mittleren physiologischen Normbereich angehoben worden. Die Hormonmessungen erfolgten in einem medizinisch-klinischen Labor mittels GC-MS.

### Beispiel 6

### Anwendung eines Avarol-Polyacrylatgels bei Psoriasis im behaarten Kopfhautbereich

Ein nach Beispiel 1 hergestelltes 5% wässriges Avarol-Polyacrylatgel wurde bei Psoriasis des behaarten Kopfbereiches bei 5 Patienten eingesetzt. Bereits wenige Tage nach der zweimal täglichen Applikation berichteten die Patienten übereinstimmend von einem Rückgang des entzündlichen Hauterscheinungsbildes, einem Nachlassen der Schuppenbildung und einer guten kosmetischen Akzeptanz der Zubereitung. Sie zog gut in die Kopfhaut ein ohne zu fetten.

### Beispiel 7

### Anwendung eines Metronidazolgels bei Entzündungen im Bereich der Zahnhälse und des Zahnfleisches

Nach Beispiel 1 unter Zusatz von 1 % Tylose statt Polyacrylatgel hergestellte wässrige Metronidazolgele [pH 5,5] zeigten bei 10 Patienten mit infizierten entzündlichen Reaktionen im Bereich von Zahnfleischtaschen sehr gute antibiotische und entzündungshemmende Wirkungen. Sie hafteten ausgezeichnet auf der Schleimhaut ohne zu reizen oder Unverträglichkeitsreaktionen auszulösen.

### Beispiel 8

### Anwendung von Gelen mit Harzen bei Entzündungen im Gelenkbereich

Wie in Beispiel 1 beschrieben wurden auch Zubereitungen mit Harzen, z.B. mit Propolis [35%, n=3] oder den Harzen der Schwedenbittermischung [20%, n=7] hergestellt. Nach Anwendung wässrig alkoholischer Polyacrylatgele beider Zubereitungen berichteten Patienten von einem Rückgang ihre entzündungsbedingten Beschwerden. Die pharmazeutisch-technologische Zubreitung wurde als angenehm empfunden, zog gut ein ohne die sonst üblichen Verfärbungen der Bekleidung zu verursachen.

## Patentansprüche

1. Verfahren zur Verbesserung der Wasserlöslichkeit von lipophilen, wirksamen Substanzen, ausgewählt aus Hormonen, Hormonanaloga, Terpenen, Harzen, Imidazolen und Derivaten hiervon, **dadurch gekennzeichnet, dass** man die lipophile(n) Substanz(en) in eine mindestens äquivalente Menge eines Lösungsmittels, bezogen auf das Gewicht der Wirksubstanz, ausgewählt aus C₁-C₁₂-aliphatischen oder C₁-C₁₂-aliphatisch-aromatischen einwertigen oder aus C₁-C₁₂- aliphatischen zweiwertigen Alkoholen oder Mischungen hiervon, bei 20° bis 70°C unter Hinzufügen von 0,1 - 20Gew.%, bezogen auf das Gewicht der Wirksubstanz, eines oder mehrerer vesikelbildender Lipide einarbeitet.

2. Verfahren gemäß Anspruch 1, **dadurch gekennzeichnet, dass** die lipophile, wirksame(n) Substanz(en) ausgewählt ist aus Hormonen, Hormonanaloga, Terpenen und Derivaten hiervon.

3. Verfahren gemäß einem der Ansprüche 1 oder 2, **dadurch gekennzeichnet, dass** das oder die vesikelbildenden Lipide ausgewählt sind aus Phosphatidylcholin oder -serin, Sphingolipiden, Squalenen, Cholesterin und Lecithin oder Mischungen hiervon.

4. Verfahren gemäß einem der Ansprüche 1 bis 3, **dadurch gekennzeichnet, dass** das oder die vesikelbildenden Lipide in einer Menge von 0,5-15 Gewichtsprozent, bezogen auf die Wirksubstanz, eingesetzt werden.

5. Verfahren zur Herstellung von wasserhaltigen Gelen mit einem Gehalt von 2 bis 90% an lipophilen Wirksubstanzen, **dadurch gekennzeichnet, dass** man die Wirksubstanz(en) in eine mindestens äquivalente Menge, bezogen auf das Gewicht der Wirksubstanz, eines Lösungsmittels, ausgewählt aus einwertigen C₁-C₁₂-atiphatischen oder C₁-C₁₂- aliphatisch- aromatischen oder C₁-C₁₂-zweiwertigen Alkoholen oder Mischungen hiervon, bei 20° bis 70°C Hinzufügen von 0,1 -20 Gew.%, bezogen auf das Gewicht der Wirksubstanz(en), eines oder mehrerer vesikelbildender Lipide einarbeitet, sodann die 1 bis 10fache Menge (Gewichtsprozent), bezogen auf das Gewicht der alkoholischen Lösung des lipophilen Wirkstoffs, einer wässrigen Lösung, enthaltend 0,1-5% eines Gelbildners, ausgewählt aus Polyacrylaten, Hydroxypropylmethyl-cellulose,Hydroxyethyl- oder-propylcellulose, Agarose, Kollagen oder anderen polymeren Kohlehydraten oder Proteinen hinzufügt und die Mischung anschließend, sofern erforderlich, abkühlt.

6. Verfahren gemäß Anspruch 5, **dadurch gekennzeichnet, dass** als Wirkstoff Steroidhormone, ausgewählt aus Estrogenen, Progesteronen und Androgenen, insbesondere Testosteron, Testolacton, Estradiol, Estriol, Dihydroepiandrosteron (DHEA), Progesteron, sowie Mischungen hiervon und deren pharmazeutisch verträglichen Estern und anderen Derivaten eingesetzt werden.

7. Verfahren gemäß Anspruch 5 oder 6, **dadurch gekennzeichnet, dass** der oder die Alkohole ausgewählt sind aus einwertigen C₁-C₈- aliphatischen Alkoholen und Mischungen hiervon mit C₁-C₈-zweiwertigen Alkoholen.

8. Verfahren gemäß einem der Ansprüche 5 bis 7, **dadurch gekennzeichnet, dass** das oder die vesikelbildenden Lipide ausgewählt sind aus Lecithin, Cholesterin, Phosphatidylcholin oder -serin, Sphingolipiden, Squalen oder Mischungen hiervon.

9. Verfahren gemäß einem der Ansprüche 5 bis 8, **dadurch gekennzeichnet, dass** das oder die vesikelbildenden Lipide in einer Menge von 0,5-15 Gewichtsprozent eingesetzt werden.

10. Verfahren gemäß einem der Ansprüche 5 bis 9, **dadurch gekennzeichnet, dass** als Gelbildner Polyacrylat eingesetzt wird.

11. Verfahren gemäß einem der Ansprüche 5 bis 10, **dadurch gekennzeichnet, dass** 10 bis 90 Gewichtsprozent an lipophilern Wirkstoff enthalten sind.

12. Verfahren gemäß einem der Ansprüche 5 bis 11, **dadurch gekennzeichnet, dass** man das erhaltene Gel in an sich bekannter Weise gefriertrocknet bei Temperaturen unter -10°C und ein lagerbeständiges wirkstoffhaltiges Pulver erhält.

13. Wirkstoffhaltiges Produkt, erhalten nach dem Verfahren gemäß einem der Ansprüche 5 bis 12, enthaltend 2 bis 90 Gewichtsprozent eines lipophilen Wirkstoffs, ausgewählt aus Hormonen, Hormonanaloga, Terpenen, Imidazolen und pharmazeutisch verträglichen Derivaten hiervon.

14. Produkt gemäß Anspruch 13, **dadurch gekennzeichnet, dass** der Wirkstoff ein oder mehrere Steroidhormon(e), ausgewählt aus Estrogenen, Progesteronen, Androgenen ist.

15. Produkt gemäß einem der Ansprüche 13 oder 14, **dadurch gekennzeichnet**, zeichnet, dass es gefriergetrocknet ist und 10 bis 90 Gewichtsprozent an Wirkstoff enthält.

16. Hochkonzentrierte Zubereitungen, erhalten gemäß Anspruch 5, **dadurch gekennzeichnet, dass** sie direkt als Gel topisch applizierbar ist.

17. Verwendung eines Wirkstoffproduktes gemäß einem der Ansprüche 13 bis 16 in der Kosmetik.

18. Verwendung eines Wirkstoffproduktes, hergestellt nach dem Verfahren gemäss einem der Ansprüche 5-12 oder eines Produktes gemäß einem der Ansprüche 13 bis 16 zur Herstellung eines kosmetischen, medizinischen oder pharmazeutischen Mittels für die topische, parenterale intra- / subcutane, intravenöse, intramuskuläre, orale oder rektale Anwendung, bzw. als Implantat.

19. Verwendung gemäß Anspruch 18, **dadurch gekennzeichnet, dass** man ein Produkt gemäß Anspruch 15 in Wasser, Alkohol, physiologischer Kochsalzlösung oder anderen geeigneten pharmazeutisch zulässigen Trägern oder Mischungen hiervon aufnimmt und die für die gewünschte Anwendung geeignete Zubereitung in an sich bekannter Weise herstellt.

20. Verwendung gemäß Anspruch 19, **dadurch gekennzeichnet, dass** man eine topische Zubereitung in Form eines Gels, einer Creme oder einer Salbe herstellt.

21. Verwendung gemäß einem der Ansprüche 18 bis 20 zur Herstellung eines Mittels zur Vorbeugung oder Behandlung von Hautalterungserscheinungen, Depressionen, Entzündungen, Arteriosklerose, Störungen des zentralen Nervensystems, Kardiovaskuläre Erkrankungen, Arthritis, degenerative Erkrankungen des Bewegungsapparates oder Infektionen.

## Claims

1. Process for improving the water solubility of lipophilic active substances selected from hormones, hormone analogues, terpenes, resins, imidazoles and derivatives thereof, **characterized in that** the lipophilic substance(s) is (are) incorporated into an at least equivalent amount, based on the weight of the active substance, of a solvent selected from C₁-C₁₂ aliphatic and C₁-C₁₂ aliphatic-aromatic monohydric alcohols, C₁-C₁₂ aliphatic dihydric alcohols and mixtures thereof, at 20° to 70°C, with the addition of 0.1 - 20 wt.%, based on the weight of the active substance, of one or more vesicle-forming lipids.

2. Process according to Claim 1, **characterized in that** the lipophilic active substance(s) is (are) selected from hormones, hormone analogues, terpenes and derivatives thereof.

3. Process according to Claim 1 or 2, **characterized in that** the vesicle-forming lipid(s) is (are) selected from phosphatidylcholine or phosphatidylserine, sphingolipids, squalenes, cholesterol, lecithin and mixtures thereof.

4. Process according to one of Claims 1 to 3, **characterized in that** the vesicle-forming lipid(s) is (are) used in an amount of 0.5 - 15 percent by weight, based on the active substance.

5. Process for the preparation of water-containing gels containing 2 to 90% of lipophilic active substances, **characterized in that** the active substance(s) is (are) incorporated into an at least equivalent amount, based on the weight of the active substance, of a solvent selected from C₁-C₁₂ aliphatic and C₁-C₁₂ aliphatic-aromatic monohydric alcohols, C₁-C₁₂ dihydric alcohols and mixtures thereof, at 20° to 70°C, with the addition of 0.1 - 20 wt.%, based on the weight of the active substance(s), of one or more vesicle-forming lipids, after which 1 to 10 times the amount (percent by weight), based on the weight of the alcoholic solution of the lipophilic active substance, of an aqueous solution containing 0.1 - 5% of a gelling agent selected from polyacrylates, hydroxypropyl methyl cellulose, hydroxyethyl or hydroxypropyl cellulose, agarose, collagen, and other polymeric carbohydrates or proteins is added, and the mixture is then cooled if necessary.

6. Process according to Claim 5, **characterized in that** the active substances used are steroid hormones selected from oestrogens, progesterones and androgens, especially testosterone, testolactone, oestradiol, oestriol, dihydroepiandrosterone (DHEA), progesterone, mixtures thereof and their pharmaceutically acceptable esters and other derivatives.

7. Process according to Claim 5 or 6, **characterized in that** the alcohol(s) is (are) selected from C₁-C₈ aliphatic monohydric alcohols and mixtures thereof with C₁-C₈ dihydric alcohols.

8. Process according to one of Claims 5 to 7, **characterized in that** the vesicle-forming lipid(s) is (are) selected from lecithin, cholesterol, phosphatidylcholine or phosphatidylserine, sphingolipids, squalene and mixtures thereof.

9. Process according to one of Claims 5 to 8, **characterized in that** the vesicle-forming lipid(s) is (are) used in an amount of 0.5 - 15 percent by weight.

10. Process according to one of Claims 5 to 9, **characterized in that** the gelling agent used is polyacrylate.

11. Process according to one of Claims 5 to 10, **characterized in that** 10 to 90 percent by weight of lipophilic active substance is present.

12. Process according to one of Claims 5 to 11, **characterized in that** the gel obtained is freeze-dried in a manner known per se at temperatures below -10°C to give a powder containing active substance that is stable on storage.

13. Product containing active substance, obtained by the process according to one of Claims 5 to 12, containing 2 to 90 percent by weight of a lipophilic active substance selected from hormones, hormone analogues, terpenes, imidazoles and pharmaceutically acceptable derivatives thereof.

14. Product according to Claim 13, **characterized in that** the active substance is one or more steroid hormones selected from oestrogens, progesterones and androgens.

15. Product according to Claim 13 or 14, **characterized in that** it is freeze-dried and contains 10 to 90 percent by weight of active substance.

16. Highly concentrated formulation obtained according to Claim 5, **characterized in that** it is directly applicable topically as a gel.

17. Use of an active product according to one of Claims 13 to 16 in cosmetics.

18. Use of an active product prepared by the process according to one of Claims 5 - 12, or of a product according to one of Claims 13 to 16, for the preparation of a cosmetic, medicinal or pharmaceutical composition for topical, parenteral, intracutaneous/ subcutaneous, intravenous, intramuscular, oral or rectal administration or as an implant.

19. Use according to Claim 18, **characterized in that** a product according to Claim 15 is taken up in water, alcohol, physiological saline or other suitable, pharmaceutically acceptable excipients, or mixtures thereof, and the formulation appropriate for the desired form of administration is prepared in a manner known per se.

20. Use according to Claim 19, **characterized in that** a topical formulation is prepared in the form of a gel, a cream or an ointment.

21. Use according to one of Claims 18 to 20 for the preparation of a composition for the prevention or treatment of skin ageing manifestations, depressions, inflammations, arteriosclerosis, disorders of the central nervous system, cardiovascular diseases, arthritis, degenerative diseases of the locomotor system or infections.

## Revendications

1. Procédé pour l'amélioration de la solubilité dans l'eau de substances actives lipophiles, choisies parmi des hormones, des analogues d'hormones, des terpènes, des résines, des imidazoles et des dérivés de ceux-ci, **caractérisé en ce que** l'on intègre la ou les substances lipophiles entre 20 °C et 70 °C dans une quantité d'un solvant au moins équivalente, rapportée au poids de la substance active, choisi parmi des alcools monovalents aliphatiques en C₁ à C₁₂ ou aliphatiques-aromatiques en C₁ à C₁₂ ou des alcools divalents aliphatiques en C₁ à C₁₂, ou des mélanges de ceux-ci, moyennant l'addition de 0,1 à 20 % en poids, rapportés au poids de la substance active, d'un ou de plusieurs lipides formant des vésicules.

2. Procédé selon la revendication 1, **caractérisé en ce que** la ou les substances lipophiles actives sont choisies parmi des hormones, des analogues d'hormones, des terpènes, et des dérivés de ceux-ci.

3. Procédé selon la revendication 1 ou 2, **caractérisé en ce que** le ou les lipides formant des vésicules sont choisis parmi la phosphatidylcholine ou -sérine, des sphingolipides, des squalènes, le cholestérol et la lécithine, ou des mélanges de ceux-ci.

4. Procédé selon l'une quelconque des revendications 1 à 3, **caractérisé en ce que** le ou les lipides formant des vésicules sont utilisés en une quantité de 0,5 à 15 pour cent en poids, rapportés à la substance active.

5. Procédé de production de gels contenant de l'eau avec une teneur de 2 à 90 % de substances actives lipophiles, **caractérisé en ce que** l'on intègre la ou les substances lipophiles actives entre 20 °C et 70 °C dans une quantité au moins équivalente, rapportée au poids de la substance active, d'un solvant choisi parmi des alcools monovalents aliphatiques en C₁ à C₁₂ ou aliphatiques-aromatiques en C₁ à C₁₂ ou des alcools divalents aliphatiques en C₁ à C₁₂, ou des mélanges de ceux-ci, moyennant l'addition de 0,1 à 20 % en poids, rapportés au poids de la substance active, d'un ou de plusieurs lipides formant des vésicules, **en ce que** l'on ajoute ensuite une solution aqueuse en quantité égale à 1 à 10 fois (pour cent en poids), rapportée au poids de la solution alcoolique de la substance active lipophile, solution aqueuse contenant 0,1 à 5% d'un gélifiant, choisi parmi des polyacrylates, l'hydroxypropylméthylcellulose, l'hydroxyéthyl- ou -propylcellulose, l'agarose, le collagène ou d'autres hydrates de carbone ou protéines, et **en ce que** l'on refroidit ensuite le mélange si nécessaire.

6. Procédé selon la revendication 5, **caractérisé en ce que**, comme substance active, on utilise des hormones stéroïdiennes, choisies parmi des oestrogènes, des progestérones et des androgènes, en particulier de la testostérone, de la testolactone, de l'oestradiol, de l'oestriol, de la dihydroépiandrostérone (DHEA), de la progestérone, ainsi que des mélanges de ceux-ci et leurs esters et autres dérivés pharmacologiquement acceptables.

7. Procédé selon la revendication 5 ou 6, **caractérisé en ce que** le ou les alcools sont choisis parmi des alcools aliphatiques monovalents en C₁ à C₈ et des mélanges de ceux-ci avec des alcools aliphatiques divalents en C₁ à C₈.

8. Procédé selon l'une quelconque des revendications 5 à 7, **caractérisé en ce que** le ou les lipides formant des vésicules sont choisis parmi la lécithine, le cholestérol, la phosphatidylcholine ou -sérine, des sphingolipides, le squalène ou des mélanges de ceux-ci.

9. Procédé selon l'une quelconque des revendications 5 à 8, **caractérisé en ce que** le ou les lipides formant des vésicules sont utilisés en une quantité de 0,5 à 15 pour cent en poids.

10. Procédé selon l'une quelconque des revendications 5 à 9, **caractérisé en ce que** du polyacrylate est utilisé comme gélifiant.

11. Procédé selon l'une quelconque des revendications 5 à 10, **caractérisé en ce que** 10 à 90 pour cent en poids de substance active lipophile sont contenus.

12. Procédé selon l'une quelconque des revendications 5 à 11, **caractérisé en ce que** l'on lyophilise le gel obtenu à des températures inférieures à-10°C de la façon connue en soi et que l'on obtient une poudre résistant au stockage contenant de la substance active.

13. Produit contenant de la substance active, obtenu d'après le procédé selon l'une quelconque des revendications 5 à 12, contenant 2 à 90 % en poids d'une substance active lipophile, choisie parmi des hormones, des analogues d'hormones, des terpènes, des imidazoles et des dérivés pharmacologiquement acceptables de ceux-ci.

14. Produit selon la revendication 13, **caractérisé en ce que** la substance active est une ou plusieurs hormones stéroïdiennes, choisies parmi des oestrogènes, des progestérones, des androgènes.

15. Produit selon l'une quelconque des revendications 13 ou 14, **caractérisé en ce qu'**il est lyophilisé et contient de 10 à 90 % en poids de substance active.

16. Préparations hautement concentrées, obtenues selon la revendication 5, **caractérisées en ce qu'**elles sont directement applicables topiquement comme gel.

17. Utilisation d'un produit de substance active selon l'une quelconque des revendications 13 à 16 en cosmétique.

18. Utilisation d'un produit de substance active, préparé d'après le procédé selon l'une quelconque des revendications 5 à 12, ou d'un produit selon l'une quelconque des revendications 13 à 16, à la préparation d'un produit cosmétique, médical ou pharmaceutique pour l'application topique, parentérale intra- / sous-cutanée, intraveineuse, intramusculaire, orale ou rectale, respectivement comme implant.

19. Utilisation selon la revendication 18, **caractérisée en ce que** l'on met un produit selon la revendication 15 dans de l'eau, de l'alcool, une solution physiologique de chlorure de sodium ou d'autres supports appropriés pharmacologiquement acceptables ou des mélanges de ceux-ci et que l'on fabrique la préparation appropriée pour l'application désirée de la façon connue en soi.

20. Utilisation selon la revendication 19, **caractérisée en ce que** l'on produit une préparation topique sous la forme d'un gel, d'une crème ou d'une pommade.

21. Utilisation selon l'une quelconque des revendications 18 à 20 à la préparation d'un produit pour la prévention ou pour le traitement de phénomènes de vieillissement de la peau, de dépressions, d'inflammations, d'artériosclérose, de troubles du système nerveux central, d'affections cardiovasculaires, d'arthrite, d'affections dégénératives de l'appareil locomoteur ou d'infections.
